# EUROPÄISCHE PATENTANMELDUNG

(11) **EP 1 134 227 A2**
(43) Veröffentlichungstag der Anmeldung: **19.09.2001**
(21) Anmeldenummer: 01113772.6
(22) Anmeldetag: 08.09.1997
(51) Int. Cl.: C07F 17/00, C08F 10/00

(54) **Methylenverbrückte Biscyclopentadienylverbindung**

(30) Priorität: 16.09.1996 DE 19637669
(62) Teilanmeldung aus: 97115504.9
(71) Anmelder: Basell Polyolefine GmbH, 77694 Kehl (DE)
(72) Erfinder: Küber, Frank, Dr., 61440 Oberursel (DE); Riedel, Michael, Dr., 46147 Oberhausen (DE); Schiemenz, Berthold, Dr., 65929 Frankfurt (DE)
(74) Vertreter: Seelert, Stefan, Dr.

(57) **Zusammenfassung**

Die vorliegende Erfindung betrifft ein Verfahren zur Herstellung einer methylenverbrückten Biscyclopentadienylverbindungen welche als katalysator komponente, z.B. für die Herstellung von Polyolefinen, eingesetzt werden können.

## Beschreibung

Die vorliegende Erfindung bezieht sich auf ein Verfahren zur Herstellung einer methylenverbrückten Biscyclopentadienylverbindung und die Verwendung dieses Verfahrens als Teilschritt bei der Herstellung eines methylenverbrückten Biscyclopentadienyl-Metallocens, welches als Katalysatorkomponente, z.B. für die Herstellung von Polyolefinen, eingesetzt werden kann.

Aus der Literatur ist die Herstellung von Polyolefinen in Gegenwart von Metallocenen in Kombination mit Aluminoxanen oder anderen Cokatalysatoren, die aufgrund ihrer Lewis-Acidität das neutrale Metallocen in ein Kation überführen und stabilisieren können, bekannt.

Metallocene und Halbsandwichkomplexe sind nicht nur hinsichtlich der Polymerisation oder Oligomerisation von Olefinen von großem Interesse, sie können auch als Hydrier-, Epoxidations-, Isomerisierungs-, C-C-Kupplungskatalysatoren eingesetzt werden (Chem. Rev. 1992, 92, 965-994).

Aus US-A-4,892,851 und EP-A-461 566 sind kohlenstoffverbrückte Metallocene bekannt. Die Synthese methylenverbrückter Metallocene verläuft über die Darstellung des methylenverbrückten Biscyclopentadienylligandsystems, welche in mehreren Stufen durchgeführt werden muß und nur mit sehr geringen Ausbeuten verläuft, vgl. J. Am. Chem. Soc. 106, **1984**, 7451 und Helv. Chim. Acta 48, **1965,** 955.

Aus der Literatur ist bekannt, daß Cyclopentadien mit cyclischen Ketonen, unter Zusatz einer Base, direkt zu verbrücktem Biscyclopentadienylliganden umgesetzt werden kann, vgl. J. Chem. Research (S), **1992**, 162. Diese Synthese verläuft mit niedrigen Ausbeuten und bedarf anschließend einer aufwendigen chromatographischen Reinigung.

Die Aufgabe der vorliegende Erfindung besteht darin, ein einfaches und wirtschaftliches Verfahren zur Herstellung von methylenverbrückten Biscyclopentadienylverbindungen mit höheren Ausbeuten bereitzustellen.

Die Aufgabe der vorliegenden Erfindung wird durch ein Verfahren zur Herstellung einer methylenverbrückten Biscyclopentadienylverbindung gelöst, wobei ein oder zwei Cyclopentadienylverbindungen LH mit Formaldehyd in monomerer, oligomerer oder polymerer Form oder mit Formaldehyd generierenden Reagenzien in Gegenwart mindestens einer Base und mindestens eines Phasentransferkatalysators umgesetzt werden.

Die methylenverbrückte Biscyclopentadienylverbindung weist die Formel I auf 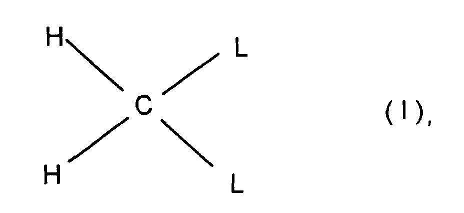 worin L unabhängig voneinander gleich oder verschieden eine Cyclopentadienylgruppe sind.

Die Cyclopentadienylgruppen L in Formel I können unsubstituiert oder substituiert sein z.B. mit C₁-C₂₀-kohlenstoffhaltigen Resten R wie C₁-C₂₀-Alkyl, C₆-C₂₀-Aryl, C₆-C₂₀-Alkylaryl, C₇-C₂₀-Arylalkyl oder C₂-C₂₀-Alkenyl. Die Reste R können auch ein Ringsystem bilden. Die Cyclopentadienylgruppen L sind gleich oder verschieden, bevorzugt gleich.

### Beispiele für substituierte Cyclopentadienylgruppen L sind:

Tetramethylcyclopentadien, 3-Methylcyclopentadien, 3-Tert.butylcyclopentadien, Methyl-tert.butylcyclopentadien, Isopropylcyclopentadien, Dimethylcyclopentadien, Trimethylcyclopentadien, Trimethylsilylcyclopentadien, Trimethylethylcyclopentadien, 3-Phenylcyclopentadien, Diphenylcyclopentadien, Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenaphthinden, 2-Methyl-4,6-diisopropylinden, Fluoren, 2-Methylfluoren oder 2,7-Di-tert.butylfluoren.

Bevorzugt sind eine oder beide Cyclopentadienylgruppen L eine substituierte Cyclopentadienylgruppe, insbesondere ein Indenderivat wie Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenaphthinden oder 2-Methyl-4,6-diisopropylinden, oder ein Fluorenylderivat wie Fluoren, 2-Methylfluoren oder 2,7-Di-tert.butylfluoren.

Beispiele für methylenverbrückte Biscyclopentadienylverbindungen der Formel I sind:
Bisindenyl-methan, Bis(2-methylindenyl)methan, Bis(2-methyl-4-phenylindenyl)methan, Bis(2-ethyl-4-phenylindenyl)methan, Bis(2-methyl-4-naphthylindenyl)methan, Bis(2-methyl-4,5-benzoindenyl)methan, Bis(methylcyclopentadienyl))methan, Biscyclopentadienyl-methan, Cyclopentadienyl-fluorenyl-methan, (3-Methylcyclopentadienyl)-fluorenyl)methan, Indenyl-fluorenyl-methan, Cyclopentadienyl-indenyl-methan, 3-Tert.butylcyclopentadienyl-fluorenyl-methan.

Zur Herstellung von Biscyclopentadienylverbindungen der Formel I, in denen die beiden Cyclopentadienylgruppen L gleich sind, wird eine Cyclopentadienylverbindung LH eingesetzt. Zur Herstellung von Biscyclopentadienylverbindungen der Formel I, in denen die beiden Cyclopentadienylgruppen L verschieden sind, werden zwei voneinander verschiedene Cyclopentadienylverbindungen LH eingesetzt. Die in dem erfindungsgemäßen Verfahren eingesetzten Cyclopentadienylverbindungen LH können substituiert oder unsubstituiert sein z.B. mit C₁-C₂₀-kohlenstoffhaltigen Resten R wie C₁-C₂₀-Alkyl, C₆-C₂₀-Aryl, C₆-C₂₀-Alkylaryl, C₇-C₂₀-Arylalkyl oder C₂-C₂₀-Alkenyl. Die Reste R können auch ein Ringsystem bilden.

### Beispiele für substituierte Cyclopentadienylverbindungen LH sind:

Tetramethylcyclopentadien, Methylcyclopentadien, Tert.butylcyclopentadien, Methyl-tert.butylcyclopentadien, Isopropylcyclopentadien, Dimethylcyclopentadien, Trimethylcyclopentadien, Trimethylsilylcyclopentadien, Trimethylethylcyclopentadien, Phenylcyclopentadien, Diphenylcyclopentadien, Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenaphthinden, 2-Methyl-4,6-diisopropylinden, Fluoren, 2-Methylfluoren oder 2,7-Di-tert.butylfluoren.

Bevorzugt sind eine oder beide der in dem erfindungsgemäßen Verfahren eingesetzten Cyclopentadienylverbindungen LH eine substituierte Cyclopentadienylverbindung, insbesondere ein Indenderivat wie Inden, 2-Methylinden, 2-Ethylinden, 3-Methylinden, 3-Tert.butylinden, 3-Trimethylsilylinden, 2-Methyl-4-phenylinden, 2-Ethyl-4-phenylinden, 2-Methyl-4-naphthylinden, 2-Methyl-4-isopropylinden, Benzoinden, 2-Methyl-4,5-benzoinden, 2-Methyl-α-acenaphthylinden oder 2-Methyl-4,6-diisopropylinden, oder ein Fluorenylderivat wie Fluoren, 2-Methylfluoren oder 2,7-Di-tert.butylfluoren.

Die in dem erfindungsgemäßen Verfahren eingesetzten Formaldehydverbindungen sind vorzugsweise Formaldehyd, Paraformaldehyd, Formalin oder Formaldehyd generierende Reagenzien wie z.B. Urotropin.

Als Base z.B. können Hydroxide der Gruppe la, IIa oder IIIa des Periodensystems der Elemente wie LiOH, NaOH, KOH, RbOH, Mg(OH)₂, Ca(OH)₂ und Sr(OH)₂ eingesetzt werden. Bevorzugt wird genau eine Base eingesetzt, z.B. LiOH, NaOH oder KOH.

Als Phasentransferkatalysator können quartäre Ammoniumsalze und Phosphoniumsalze der allgemeinen Form [R³₄Z]⁺X⁻ eingesetzt werden, wobei R³ gleich oder verschieden sind und ein Wasserstoffatom, oder eine C₁-C₄₀-kohlenstoffhaltige Gruppe wie eine C₁-C₂₀-Alkyl-, eine C₁-C₁₀-Alkoxy-, eine C₆-C₂₀-Aryl-, eine C₂-C₁₂-Alkenyl-, ein C₇-C₄₀-Arylalkyl-, eine C₇-C₄₀-Alkylaryl-, oder eine C₈-C₄₀-Arylalkenylgruppe bedeuten, die jeweils Reste wie -NR⁴₃, -SR⁴₂, -SiR⁴₃ oder -OSiR⁴₃ tragen können, worin R⁴ gleich oder verschieden ein Halogenatom, eine C₁-C₁₀ Alkylgruppe oder eine C₆-C₁₀ Arylgruppe sind, oder zwei oder mehr Reste R³ zusammen mit den sie verbindenen Atomen ein Ringsystem bilden können, welches bevorzugt 4 bis 40, besonders bevorzugt 5 bis 15 Kohlenstoffatome enthält, Z gleich Stickstoff oder Phosphor bedeutet und X⁻ gleich ein Halogenid, Hydroxid, Tetrahalogenborat, (z.B. Tetrafluoroborat), Hydrogensulfat, Sulfat oder Hexahalogenphosphat, (z.B. Hexafluorophosphat) ist.

Beispiele für als Phasentransferkatalysatoren geeignete Verbindungen sind:
Benzyltrimethylammoniumchlorid,
Benzyltrimethylammoniumhydroxid (insbesondere als wäßrige 40%-ige Lösung), Hexadecyltrimethylammoniumbromid,
Hexadecyltrimethylammoniumchlorid (insbesondere als wäßrige 50%-ige Lsg.), Ethylhexadecyldimethylammoniumbromid,
Tetraethylammoniumtetrafluoroborat,
Tetraethylammoniumbromid,
Tetraethylammoniumhydroxid (insbesondere als wäßrige 20%-ige Lösung), Benzyltriethylammoniumchlorid,
Benzyltriethylammoniumhydroxid,
Tetrapropylammoniumbromid,
Tetrabutylammoniumchlorid,
Tetrabutylammoniumfluorid-trihydrat,
Tetrabutylammoniumtetrafluoroborat,
Tetrabutylammoniumhydrogensulfat,
Tetrabutylammoniumhydroxid (insbesondere als 12,5%-ige Lösung in Methanol) Benzyltributylammoniumbromid,
Tetraoctylammoniumbromid,
Methyltrioctylammoniumchlorid,
Tetrabutylphosphoniumbromid,
Tetrabutylphosphoniumchlorid,
Tributylhexadecylphosphoniumbromid,
Ethyltrioctylphosphoniumbromid,
Butyltriphenylphosphoniumchlorid
und Tetraphenylphosphoniumbromid.

Weiterhin können als Phasentransferkatalysatoren Kronenverbindungen, insbesondere solche der allgemeinen Formeln II, III und IV eingesetzt werden, 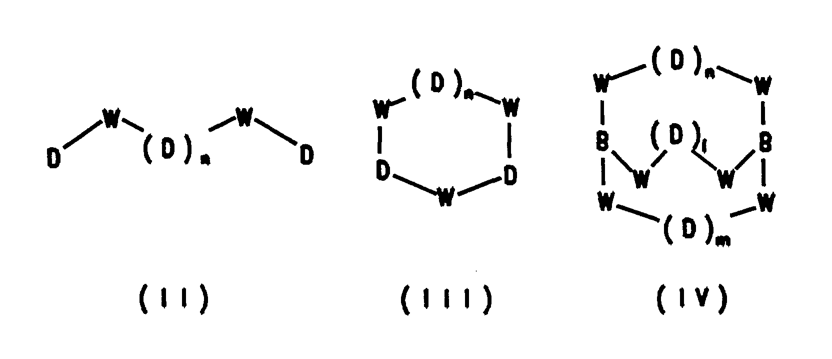 worin
- D: gleich S, O, NR⁵, PR⁵ ist und R⁵ gleich oder verschieden sind und ein Wasserstoffatom, ein Halogenatom, eine C₁-C₄₀-kohlenstoffhaltige Gruppe wie eine C₁-C₂₀-Alkyl-, eine C₁-C₁₀-Alkoxy-, eine C₆-C₂₀-Aryl-, eine C₂-C₁₂-Alkenyl-, ein C₇-C₄₀-Arylalkyl-, eine C₇-C₄₀-Alkylaryl- oder eine C₈-C₄₀-Arylalkenylgruppe bedeutet, die jeweils Reste -NR⁶₃, -SR⁶₂, -SiR⁶₃ oder -OSiR⁶₃ tragen können, worin R⁶ gleich oder verschieden ein Halogenatom, eine C₁-C₁₀ Alkylgruppe oder eine C₆-C₁₀ Arylgruppe sind,
- W: gleich oder verschieden [R⁷₂C]ₙ ist, wobei R⁷ gleich oder verschieden sind, und ein Wasserstoffatom, ein Halogenatom, eine C₁-C₄₀-kohlenstoffhaltige Gruppe wie eine C₁-C₂₀-Alkyl-, eine C₁-C₁₀-Alkoxy-, eine C₆-C₂₀-Aryl-, eine C₂-C₁₂-Alkenyl-, ein C₇-C₄₀-Arylalkyl-, eine C₇-C₄₀-Alkylaryl-, oder eine C₈-C₄₀-Arylalkenylgruppe bedeutet, die jeweils Reste -NR⁸₃, -SR⁸₂, -SiR⁸₃ oder -OSiR⁸₃ tragen können, worin R⁸ ein Halogenatom, eine C₁-C₁₀ Alkylgruppe oder eine C₆-C₁₀ Arylgruppe ist, oder zwei oder mehr Reste R⁷ zusammen mit den sie verbindenden Atomen ein Ringsystem bilden können, welches bevorzugt 4 bis 40, besonders bevorzugt 5 bis 15 Atome, insbesondere Kohlenstoffatome, enthält,
- n, l und m: gleich oder verschieden, eine ganze Zahl von 1 bis 40, bevorzugt 1 bis 5 sind, und bevorzugt gleich sind, und
- B: gleich oder verschieden ein Element der V. Hauptgruppe des Periodensystems wie Stickstoff oder Phosphor ist.

Beispiele für Kronenetherverbindungen sind:
12-Krone-4, 15-Krone-5, Benzo-15-Krone-5, 18-Krone-6, Decyl-18-Krone-6, Dibenzo-18-Krone-6, Dicyclohexyl-18-Krone-8, Dibenzo-24-Krone-8, (+)-18-Krone-6-tetracarbonsäure, N-Phenyl-aza-15-Krone-5, ® Kryptofix 21, ® Kryptofix 22, ® Kryptofix 22 DD, ® Kryptofix 23, Tris[2-(-methoxyethoxy)-ethyl]amin, ® Kryptofix 5, ® Kryptofix 111, ® Kryptofix 211, ® Kryptofix 221, ® Kryptofix 221 D, ® Kryptofix 222, ® Kryptofix 222 B (50%-ige Lösung in Toluol), ® Kryptofix 222 BB, ® Kryptofix 222 CC (50%-ige Lösung in Toluol), ® Kryptofix 222 D (50%-ige Lösung in Toluol), ® Kryptofix 221 B (Polymer), und ® Kryptofix 222 B (Polymer).

In dem erfindungsgemäßen Verfahren wird mindestens ein Phasentransferkatalysator eingesetzt. Die Konzentration des Phasentransferkatalysators kann 0,1 bis 100 Mol-%, bezogen auf die eingesetzte Menge an Cyclopentadienylverbindung(en) LH, besonders bevorzugt 1 bis 20 Mol%, betragen.

Das erfindungsgemäße Verfahren wird in einem Ein- oder Mehrphasensystem, in Gegenwart mindestens einer Base und mindestens eines Phasentransferkatalysators durchgeführt. Bevorzugt wird das erfindungsgemäße Verfahren in einem Mehrphasensystem, insbesondere in einem Zweiphasensystem durchgeführt, wobei eine Phase ein organisches Lösemittel z.B. ein aromatisches Lösemittel wie Toluol, Xylol oder ein aliphatisches Lösemittel wie Tetrahydrofuran, Hexan oder Dichlormethan darstellt und die zweite Phase Wasser ist. Insbesondere sind Zweiphasensysteme Toluol/Wasser, Dichlormethan/Wasser und Tetrahydrofuran/Wasser bevorzugt. Die Konzentration an Base in der wäßrigen Phase kann zwischen 5- und 70 Gew.-% betragen, bevorzugt 25 bis 60 Gew.-%.

Zur Synthese von methylenverbrückten Biscyclopentadienylverbindungen mit zwei gleichen Cyclopentadienylgruppen L kann die Cyclopentadienylverbindung LH im Überschuß (bezogen auf die Formaldehydverbindung) eingesetzt werden, bevorzugt werden 2 bis 3 Äquivalente der Cyclopentadienylverbindung LH, bezogen auf das eingesetzte Formaldehyd verwendet. Bei der Synthese von methylenverbrückten Biscyclopentadienylverbindungen mit zwei voneinander verschiedenen Cyclopentadienylgruppen L, werden zwei voneinander verschiedene Cyclopentadienylverbindungen LH eingesetzt. Dabei wird zuerst eine der beiden Cyclopentadienylverbindungen mit Formaldehyd zur Reaktion gebracht, wobei das Verhältnis der beiden Komponenten ungefähr 1 : 1 beträgt. Nach einer Reaktionszeit, die zwischen 30 min und 100 h, vorzugsweise zwischen 30 min und 20 h liegen kann, erfolgt die Zugabe der zweiten Cyclopentadienylverbindung.

Die Reaktionstemperatur kann zwischen 0 °C und 100 °C betragen, bevorzugt 0 °C bis 30 °C. Die Reaktionszeiten liegen in der Regel zwischen 30 min und 100 h, vorzugsweise zwischen 30 min und 20 h.

Das Volumenverhältnis Organische Phase/Wasser (z.B. Toluol/Wasser, Dichlormethan/Wasser oder Tetrahydrofuran/Wasser) kann zwischen 10000:1 und 1:50, bevorzugt zwischen 100:1 und 1:10, besonders bevorzugt zwischen 10:1 und 1:1 liegen.

Bevorzugt wird eine Mischung der Cyclopentadienylverbindung LH und der Formaldehydverbindung in dem organischen Lösungsmittel vorgelegt und die wäßrige Phase, in der sich sowohl die Base sowie auch der Phasentransferkatalysator befindet, zudosiert. Auch die umgekehrte Reaktionsführung ist möglich, d.h. zu dem Zweiphasensystem (z.B. Toluol/Wasser, Dichlormethan/Wasser oder Tetrahydrofuran/Wasser), in dem die Cyclopentadienylverbindung LH, die Base und der Phasentranfserkatalysator enthalten sind, kann Formaldehyd über einen Zeitraum von 1 min bis 100 h, bevorzugt von 15 min bis 4 h, zugetropft werden.

Die mit dem erfindungsgemäßen Verfahren erhältlichen methylenverbrückten Biscyclopentadienylverbindungen können als Doppelbindungsisomere anfallen.

Das erfindungsgemäße Verfahren zeichnet sich insbesondere dadurch aus, daß methylenverbrückte Biscyclopentadienylverbindungen in einer einfachen, einstufigen Synthese in hoher Ausbeute erhalten werden können. Das Substitutionsmuster der Cyclopentadienylgruppen L kann dabei in einem weiten Bereich variiert werden.

Gegenstand der vorliegenden Erfindung ist ferner die Verwendung des erfindungsgemäßen Verfahrens als Teilschritt eines Verfahrens zur Herstellung eines methylenverbrückten Biscyclopentadienyl-Metallocens, insbesondere eines kohlenstoffverbrückten Biscyclopentadienyl-Metallocens der nachstehenden Formel V 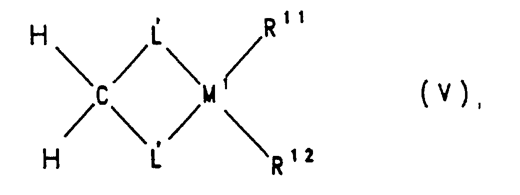 worin
- M¹: ein Element der Gruppen IIIb, IVb, Vb oder Vlb des Periodensystems der Elemente, insbesondere der Gruppe IVb ist,
- L': unabhängig voneinander gleich oder verschieden eine Cyclopentadienylgruppe sind, und
- R¹¹ und R¹²: gleich oder verschieden sind und Wasserstoff, ein Halogenatom oder einen C₁-C₄₀-kohlenstoffhaltigen Rest wie C₁-C₁₀-Alkyl, C₁-C₁₀-Alkoxy, C₆-C₁₄-Aryl, C₆-C₁₄-Aryloxy, C₂-C₁₀-Alkenyl, C₇-C₄₀-Arylalkyl, C₇-C₄₀-Alkylaryl, C₈-C₄₀-Arylalkenyl, Hydroxy, NR⁵₂, worin R⁵ ein C₁-C₁₀-Alkyl, C₁-C₁₀-Alkoxy, C₆-C₁₄-Aryl, C₆-C₁₄-Aryloxy, C₂-C₁₀-Alkenyl, C₇-C₄₀-Arylalkyl, C₇-C₄₀-Alkylaryl oder C₈-C₄₀-Arylalkenyl bedeuten.

Die Cyclopentadienylgruppen L' in Formel V können unsubstituiert oder substituiert sein z.B. mit C₁-C₂₀-kohlenstoffhaltigen Resten R wie C₁-C₂₀-Alkyl, C₆-C₂₀-Aryl, C₆-C₂₀-Alkylaryl, C₇-C₂₀-Arylalkyl oder C₂-C₂₀-Alkenyl. Die Reste R können auch ein Ringsystem bilden. Die Cyclopentadienylgruppe L sind gleich oder verschieden, bevorzugt gleich.

Beispiele für substituierte Cyclopentadienylgruppen L' sind:
Tetramethylcyclopentadienyl, 3-Methylcyclopentadienyl, 3-Tert.butylcyclopentadienyl, Methyl-tert.butylcyclopentadienyl, Isopropylcyclopentadienyl, Dimethylcyclopentadienyl, Trimethylcyclopentadienyl, Trimethylethylcyclopentadienyl, 3-Phenylcyclopentadienyl, Diphenylcyclopentadienyl, 3-Trimethylsilylcyclopentadien, Indenyl, 2-Methylindenyl, 2-Ethylindenyl, 3-Methylindenyl, 3-Tert.butylindenyl, 3-Trimethylsilylindenyl, 2-Methyl-4-phenylindenyl, 2-Ethyl-4-phenylindenyl, 2-Methyl-4-naphthylindenyl, 2-Methyl-4-isopropylindenyl, Benzoindenyl, 2-Methyl-4,5-benzoindenyl, 2-Methyl-α-acenaphthylindenyl, 2-Methyl-4,6-diisopropylindenyl, Fluorenyl, 2-Methylfluorenyl, 2,7-Diphenyl-fluorenyl oder 2,7-Di-tert.butylfluorenyl.

Bevorzugt sind eine oder beide Cyclopentadienylgruppen L' eine substituierte Cyclopentadienylgruppe, insbesondere ein Indenylderivat wie Indenyl, 2-Methylindenyl, 2-Ethylindenyl, 3-Methylindenyl, 3-Tert.butylindenyl, 3-Trimethylsilylindenyl, 2-Methyl-4-phenylindenyl, 2-Ethyl-4-phenylindenyl, 2-Methyl-4-naphthylindenyl, 2-Methyl-4-isopropylindenyl, Benzoindenyl, 2-Methyl-4,5-benzoindenyl, 2-Methyl-α-acenaphthylindenyl oder 2-Methyl-4,6-diisopropylindenyl, oder ein Fluorenylderivat wie Fluorenyl, 2-Methylfluorenyl, 2,7-Diphenyl-fluorenyl oder 2,7-Di-tert.butylfluorenyl.

Bevorzugt ist M¹ ein Element der Gruppe IV des Periodensystems der Elemente wie Titan, Zirkonium oder Hafnium, insbesondere Zirkonium. R¹ und R² sind gleich oder verschieden, bevorzugt gleich und bedeuten Wasserstoff, C₁-C₁₀-Alkyl oder C₆-C₁₄-Aryl, insbesondere C₁-C₅ Alkyl, und die Reste R¹¹ und R¹² sind bevorzugt gleich und bedeuten C₁-C₄-Alkyl wie Methyl oder ein Halogenatom wie Chlor.

Beispiele für nach dem erfindungsgemäßen Metallocen-Herstellungsverfahren erhältliche kohlenstoffverbrückte Biscyclopentadienyl-Metallocene sind:
Methylen-bis(cyclopentadienyl)zirkoniumdichlorid,
Methylen-bis(2,3,4,5-tetramethylcyclopentadienyl)zirkoniumdichlorid,
Methylen-bis(2,3,4-trimethylcyclopentadienyl)zirkoniumdichlorid,
Methylen-bis(1-indenyl)zirkoniumdichlorid,
Methylen-bis(1-(2-methylindenyl)zirkoniumdichlorid,
Methylen-bis(1-(4-phenylindenyl))zirkoniumdichlorid,
Methylen-bis(1-(4-isopropylindenyl))zirkoniumdichlorid,
Methylen-bis(1-(4-isopropylindenyl))hafniumdichlorid,
Methylen-bis(1-(4,5-benzoindenyl)zirkoniumdichlorid,
Methylen-bis(1-(4,5-benzoindenyl))hafniumdichlorid,
Methylen-(1-indenyl)-cyclopentadienyl-zirkoniumdichlorid,
Methylen-(1-indenyl)-(3-methylcyclopentadienyl)-zirkoniumdichlorid,
Methylen-(1-(4-isopropyl)indenyl)-cyclopentadienyl-zirkoniumdichlorid,
Methylen-(1-indenyl)-cyclopentadienyl-titandichlorid,
Methylen-(1-indenyl)-3-methylcyclopentadienyl-titandichlorid,
Methylen-(1-indenyl)-(9-fluorenyl)-zirkoniumdichlorid,
Methylen-(9-fluorenyl)-(3-methylcyclopentadienyl)-zirkoniumdichlorid,
Methylen-(9-fluorenyl)-(3-tert. butylcyclopentadienyl)-zirkoniumdichlorid,
Methylen-(9-fluorenyl)-cyclopentadienyl-zirkoniumdichlorid,
Methylen-(9-(2,7-di-tert.butyl)fluorenyl)-cyclopentadienyl-zirkoniumdichlorid.
Methylen-(9-(2,7-diphenyl)fluorenyl-cydopentadienyl-zirkoniumdichlorid.

Bevorzugt sind über die 1-Positionen der Indenylliganden verbrückte Bisindenyl-Verbindungen der Formel Methylen-bis (1-Ind) M¹R¹¹R¹², worin Ind ein Indenylderivat ist und M¹, R¹¹ und R¹² wie in Formel V definiert sind. Indenylderivate Ind sind z.B. Indenyl, welches C₁-C₂₀-Kohlenwasserstoffreste wie C₁-C₂₀-Alkyl, C₆-C₂₀-Aryl, C₆-C₂₀-Alkylaryl, C₆-C₂₀-Arylalkyl oder C₂-C₂₀-Alkenyl tragen kann und die auch ein Ringsystem bilden können, z.B. 2-Methylindenyl, 2-Ethylindenyl, 3-Methylindenyl, 3-Tert.butylindenyl, 3-Trimethylsilylindenyl, 2-Methyl-4-phenylindenyl, 2-Ethyl-4-phenylindenyl, 2-Methyl-4-naphthylindenyl, 2-Methyl-4-isopropylindenyl, Benzoindenyl, 2-Methyl-4,5-benzoindenyl, 2-Methyl-α-acenaphthylindenyl oder 2-Methyl-4,6-diisopropylindenyl.

Die vorliegende Erfindung betrifft auch ein Verfahren zur Herstellung eines methylenverbrückten Biscyclopentadienyl-Metallocens, enthaltend die Schritte:
a) Umsetzung einer oder zweier Cyclopentadienylverbindungen LH mit Formaldehyd in monomerer, oligomerer oder polymerer Form bzw. einem Formaldehyd generierenden Reagenz in Gegenwart mindestens einer Base und mindestens eines Phasentransferkatalysators zu einer methylenverbrückten Biscyclopentadienylverbindung, und
b) Umsetzung der in Schritt a) erhaltenen methylenverbrückten Biscyclopentadienylverbindung mit einer Metallverbindung M¹Xₚ, worin M¹ ein Element der Gruppe IIIb, IVb, Vb oder Vlb des Periodensystems der Elemente ist, X ein C₁-C₄₀-kohlenstoffhaltiger Rest, wie C₁-C₁₀-Alkyl oder NR¹³₂, worin R¹³ ein C₁-C₂₀-Kohlenwasserstoffrest wie C₁-C₁₀-Alkyl oder C₆-C₁₆-Aryl ist, ein Halogen oder ein Pseudohalogen ist und p eine ganze Zahl von 0 bis 4 ist, unter Bedingungen, unter denen die in Schritt a) erhaltene methylenverbrückte Biscyclopentadienylverbindung zum methylenverbrückten Biscyclopentadienyl-Metallocen komplexiert wird.

Der zweite Schritt (b) des Herstellungsverfahrens des kohlenstoffverbrückten Biscyclopentadienyl-Metallocens kann nach literaturbekannten Verfahren durchgeführt werden z.B. AU-A-31478/89; J. Organomet. Chem. 1988, 342, 21 oder EP-A-284 707, auf die hiermit ausdrücklich Bezug genommen wird). Bevorzugt wird die kohlenstoffverbrückte Biscyclopentadienverbindung zunächst mit einer Verbindung der Formel R¹⁴M² umgesetzt, worin M² ein Metall der Gruppe la, IIa oder IIIa des Periodensystems der Elemente bedeutet und R¹⁴ ein C₁-C₂₀-Kohlenwasserstoffrest wie C₁-C₁₀-Alkyl oder C₆-C₁₄-Aryl ist, und anschließend mit der Metallverbindung M¹Xₚ umgesetzt. Die Umsetzungen finden bevorzugt in einem geeigneten Lösemittel z.B. einem aliphatischen oder aromatischen Lösemittel, wie Hexan oder Toluol, einem etherischen Lösemittel, wie Tetrahydrofuran oder Diethylether oder in halogenierten Kohlenwasserstoffen, wie Methylenchlorid oder o-Dichlorbenzol statt. In der Metallverbindung der Formel M¹Xₚ ist M¹ bevorzugt ein Element der Gruppe IIIb des Periodensystems der Elemente, X ist bevorzugt ein Halogenatom oder NR¹³₂, worin R¹³ ein C₁-C₁₀-Kohlenwasserstoffrest wie C₁-C₁₀-Alkyl oder C₆-C₁₀-Aryl ist und p ist bevorzugt 4. Die kohlenstoffverbrückte Biscyclopentadienylverbindung kann als Isomerengemisch eingesetzt werden.

Methylenverbrückte Biscyclopentadienyl-Metallocenhalogenide der Formel V können nach für andere kohlenstoffverbrückte Biscyclopentadienyl-Metallocendihalogenide literaturbekannten Methoden, z.B. durch Umsetzung mit Alkylierungsmitteln, wie z.B. Lithiumalkylen, zu den entsprechenden Mono- oder Dialkylverbindungen derivatisiert werden, vgl. J. Am. Chem. Soc. 1973, 95, 6263.

Die methylenverbrückten Biscyclopentadienyl-Metallocene der Formel V können als Gemisch der racemischen Form und der meso-Form anfallen. Die Trennung der isomeren Formen, insbesondere die Abtrennung der meso-Form, ist im Prinzip bekannt, vgl. AU-A-31478/89, EP-A-284 707 und J. Organomet. Chem. 342, **1988**, 21. Die Abtrennung kann z.B. durch Extraktion oder Umkristallisation mit verschiedenen Lösemitteln erfolgen.

Das erfindungsgemäße Verfahren erlaubt mit hoher Ausbeute die einfache Herstellung von methylenverbrückten Biscyclopentadienyl-Metallocenen.

Die mit dem erfindungsgemäßen Metallocen-Herstellungsverfahren erhältlichen methylenverbrückten Biscyclopentadienyl-Metallocene können zusammen mit einem Cokatalysator als hochaktive Katalysatorkomponenten z.B. für die Herstellung von Olefinpolymeren eingesetzt werden.

Polymerisiert werden können Olefine, insbesondere solche der Formel R^{a}-CH=CH-R^{b}, worin R^{a} und R^{b} gleich oder verschieden sind und ein Wasserstoffatom oder einen Kohlenwasserstoffrest mit 1 bis 20 Kohlenstoffatomen bedeuten. R^{a} und R^{b} können auch mit den sie verbindenen Kohlenstoffatomen einen Ring bilden. Beispiele für solche Olefine sind Ethylen, Propylen, 1-Buten, 1-Hexen, 1-Octen, 4-Methyl-1-penten, 1,3-Butadien, Isopren, Norbornen, Dimethanooctahydronaphthalin oder Norbornadien. Insbesondere können Propylen und Ethylen homopolymerisiert werden, Ethylen mit einem C₃-C₂₀-Olefin und/oder einem C₄-C₂₀-Dien copolymerisiert werden oder Ethylen mit einem Cycloolefin copolymerisiert werden.

Die Polymerisation kann eine Homo- oder eine Copolymerisation sein und in Lösung, in Suspension oder in der Gasphase, kontinuierlich oder diskontinuierlich, ein- oder mehrstufig bei einer Temperatur von 0 ° C bis 200 °C, vorzugsweise 30 °C bis 100 °C durchgeführt werden.

Prinzipiell ist als Cokatalysator in der Polymerisation jede Verbindung geeignet, die aufgrund ihrer Lewis-Acidität das neutrale Metallocen in ein Kation überführen und dieses stabilisieren kann ("labile Koordination"). Darüber hinaus soll der Cokatalysator oder das aus ihm gebildete Anion keine weiteren Reaktionen mit dem gebildeten Kation eingehen, vgl. EP-A-427 697. Als Cokatalysator wird bevorzugt eine Aluminiumverbindung und/oder Borverbindung eingesetzt.

Als Cokatalysatoren werden bevorzugt Aluminoxane verwendet, vgl. EP-A-129 368 und Polyhedron 9, **1990**, 429. An Stelle oder neben eines Aluminoxans können Borverbindungen, insbesondere der Formeln R_{X}NH_{4-X}BR₄', RₓPH₄₋ₓBR₄', R₃CBR₄' oder BR₃' als Cokatalysatoren verwendet werden. In diesen Formeln bedeutet x eine ganze Zahl von 1 bis 4, bevorzugt 3, die Reste R sind gleich oder verschieden, bevorzugt gleich, und bedeuten C₁-C₁₀-Alkyl, C₆-C₁₈-Aryl oder 2 Reste R bilden zusammen mit den sie verbindenen Atomen einen Ring, und die Reste R'sind gleich oder verschieden, bevorzugt gleich, und stehen für C₆-C₁₈-Alkyl oder C₆-C₁₈-Aryl, das durch Alkyl, Haloalkyl oder Fluor substituiert sein kann, vgl. EP-A-277 003, EP-A-277 004, EP-A-426 638 und EP-A-427 697.

Es ist möglich, das Metallocen vor dem Einsatz in der Polymerisationsreaktion mit einem Cokatalysator, insbesondere einem Aluminoxan vorzuaktivieren. Dadurch kann die Polymerisationsaktivität deutlich erhöht werden. Die Voraktivierung des Metallocens wird vorzugsweise in Lösung vorgenommen. Bevorzugt wird dabei das Metallocen in eine Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Zur Entfernung von im Olefin vorhandenen Katalysatorgiften ist eine Reinigung mit einer Aluminiumverbindung, bevorzugt einem Aluminiumalkyl, wie Trimethylaluminium oder Triethylaluminium, vorteilhaft. Diese Reinigung kann sowohl im Polymerisationssystem selbst erfolgen, oder das Olefin wird vor der Zugabe in das Polymerisationssystem mit der Aluminiumverbindung in Kontakt gebracht und anschließend wieder abgetrennt.

Als Molmassenregler und/oder zur Steigerung der Katalysatoraktivität kann in dem Polymerisationsverfahren Wasserstoff zugegeben werden. Hierdurch können niedermolekulare Polyolefine wie Wachse erhalten werden.

Bevorzugt wird das Metallocen mit dem Cokatalysator außerhalb des Polymerisationsreaktors in einem separaten Schritt unter Verwendung eines geeigneten Lösemittels umgesetzt. Dabei kann eine Trägerung vorgenommen werden.

In dem Verfahren kann mit Hilfe des Metallocens eine Vorpolymerisation erfolgen. Zur Vorpolymerisation wird bevorzugt das (oder eines der) in der Polymerisation eingesetze(n) Olefin(e) verwendet.

Der zur Olefinpolymerisation eingesetzte Katalysator kann geträgert sein. Durch die Trägerung läßt sich beispielsweise die Kornmorphologie des hergestellten Polymers steuern. Dabei kann das Metallocen zunächst mit dem Träger und anschließend mit dem Cokatalysator umgesetzt werden. Es kann auch zunächst der Cokatalysator geträgert werden und anschließend mit dem Metallocen umgesetzt werden. Auch ist es möglich, das Reaktionsprodukt von Metallocen und Cokatalysator zu trägem. Geeignete Trägermaterialien sind beispielsweise Silikagele, Aluminiumoxide, festes Aluminoxan oder andere anorganische Trägermaterialien wie beispielsweise Magnesiumchlorid. Ein geeignetes Trägermaterial ist auch ein Polyolefinpulver in feinverteilter Form. Die Herstellung des geträgerten Cokatalysators kann beispielsweise wie in EP-A-567 952 beschrieben durchgeführt werden.

Vorzugsweise wird der Cokatalysator, wie Aluminoxan, auf einen Träger wie beispielsweise Silikagele, Aluminiumoxide, festes Aluminoxan oder andere anorganische Trägermaterialien wie beispielsweise Magnesiumchlorid oder auch ein Polyolefinpulver in feinverteilter Form aufgebracht und dann mit dem Metallocen umgesetzt.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Hexan, Heptan, Isooctan, Cyclohexan, Methylcyclohexan genannt. Weiterhin kann auch eine Benzin- bzw. hydrierte Dieselölfraktion benutzt werden. Brauchbar ist auch Toluol. Bevorzugt wird im flüssigen Monomeren polymerisiert.

Durch Anwendung von Wasserstoff oder durch Erhöhung der Polymerisationstemperatur sind auch Polyolefine niedriger Molmasse, wie Wachse zugänglich, deren Härte oder Schmelzpunkt durch den Comonomergehalt variiert werden können. Durch Wahl des Polymerisations-Verfahrens und der Comonomerart(en), sowie Comonomermenge(n) lassen sich Olefincopolymere mit elastomeren Eigenschaften, wie z.B. Ethylen/Propylen/1,4-Hexadien-Terpolymere herstellen.

Die vorliegende Erfindung wird anhand der nachfolgenden Beispiele näher erläutert.

### Beispiele

### Beispiel 1

### Herstellung von Bis(1-indenyl)methan

100,0 g (0,86 mol) Inden wurden in 400 ml Toluol gelöst. Anschließend wurden 86,2 g (2,2 mol) Natriumhydroxid und 19,6 g (86 mmol) Triethylbenzylammoniumchlorid zugegeben. Die Zugabe von 12,9 g (0,43 mol) Paraformaldehyd erfolgte portionsweise über einen Zeitraum von 30 min. Nach einer Reaktionszeit von 5 Stunden wurde mit 200 ml Wasser hydrolysiert. Die wäßrige Phase wurde abgetrennt und zweimal mit jeweils 100 ml Diethylether extrahiert. Die vereinigten organischen Phasen wurden über MgSO₄ getrocknet. Das Lösungsmittel wurde im Vakuum entfernt und das Rohprodukt durch Destillation im Ölpumpenvakuum gereinigt. Es wurden 84,1 g Bis(1-indenyl)methan mit 80%-iger Ausbeute in Form eines gelben Feststoffs erhalten.
¹H-NMR (200 MHz, CDCl₃): 7,6 - 7,1 (m, 8H, arom. H), 6,22 (s, 2H, Olefin, H), 3,85 (s, 2H, CH₂), 3,35 (d, 4H, CH₂). Massenspektrum: 244 M+, korrektes Zerfallsmuster.

### Beispiel 2

### Herstellung von Methylen-(bis-1-indenyl)zirkoniumdichlorid

Eine Lösung von 10 g (41 mmol) Bis-(1-indenyl)methan wurde in 50 ml Diethylether bei Raumtemperatur unter Argonschutz mit 32,8 ml (82 mmol) einer 2,5 M Butyllithiumlösung in Hexan versetzt und über Nacht gerührt. Nach Zusatz von 40 ml Hexan wurde die beigefarbene Suspension fitriert und der Rückstand mit 20 mlPentan gewaschen. Das Dilithiosalz wurde im Ölpumpenvakuum getrocknet und dann bei -78°C zu einer Suspension von 9,6 g (41 mmol) ZrCl₄ in Dichlormethan gegeben. Die Mischung wurde innerhalb 1 h auf Raumtemperatur erwärmt und noch 30 min bei dieser Temperatur gerührt. Nach Abziehen des lösemittels wurde der orangebraune Rückstand mit 50 ml Toluol extrahiert. Nach Abziehen des Lösemittels wurden 8,6 g (52 %) eines orangen Pulvers erhalten. Das Verhältnis des Racemats zur meso-Form wurde zu 2:1 bestimmt. Durch Umkristallisation aus Toluol wurden 4,1 g (25 %) des reinen Racemats erhalten.
¹H-NMR (200 MHz, CDCl₃): 7,6-7,0 (m, 8H, arom. H), 6,58 (d, 2H, Cp-H), 5,96 (d, 2H, Cp-H), 4,79 (s, 2H, CH_{2).}
Massenspektrum: 402 M+, korrektes Zerfallsmuster.

### Beispiel 3

### Herstellung von Bis(1-(2-methyl)indenyl)methan

100 g (0,77 mol) 2-Methylinden wurden in 400 ml Toluol gelöst. Anschließend wurde eine Lösung von 86,2 g (2,2 mol) Natriumhydroxid und 19,6 g (86 mmol) Triethylbenzylammoniumchlorid in 86,2 ml Wasser (50 %iger NaOH-Lösung) zugegeben. Die Zugabe von 31,2 g (0,39 mol) Formalin (37 %ig) erfolgte tropfenweise über einen Zeitraum von 30 min. Nach einer Reaktionszeit von 5 Stunden wurde mit 100 ml H₂O hydrolysiert. Die wäßrige Phase wurde abgetrennt, zweimal mit jeweils 100 ml Diethylether extrahiert und vereinigten organischen Phasen wurden über MgSO₄ getrocknet. Das Lösungsmittel wurde im Ölpumpenvakuum entfernt und das Rohprodukt bei 10⁻¹mbar destilliert. Es wurden 40,4 g Bis-(1-(2-methyl)indenyl)methan mit 38 %iger Ausbeute in Form eines gelben Feststoffs erhalten.
¹H-NMR (200 MHz, CDCl₃): 7,6-6,9 (m, 8H,arom. H), 6,27 (s, 2H, Olefin, H), 3,88 (s, 2H, CH₂), 3,37 (s, 2H, CH₂), 1,61 (s, 3H, CH₃).
Massenspektrum: 272 M+, korrektes Zerfallsmuster.

### Beispiel 4

### Herstellung von Methylen-(Bis-1-(2-methylindenyl)zirkoniumdichlorid

Eine Lösung von 11,1 g (40 mmol) Bis-(1-methyl)indenyl)methan in 50 ml Toluol und 1,6 ml (80 mmol) Diethylether wurden bei Raumtemperatur unter Argonschutz mit 30 ml (80 mmol) einer 20 %igen n-Butyllithiumlösung in Toluol versetzt und 16 h gerührt. Die erhaltene Suspension wurde auf -30°C gekühlt, dann wurden 9,3 g (40 mmol) Zirkontetrachlorid zugegeben. Die Mischung wurde innerhalb 1 h langsam auf 30°C erwärmt und noch 3 h bei dieser Temperatur gerührt. Nach Zugabe von 5 g Celite wurde filtriert und der Rückstand zweimal mit ca. 30 ml Toluol gewaschen. Die vereinigten Filtrate wurden im Ölpumpenvakuum auf 40 ml eingeengt und die konzentrierte Lösung auf -30°C gekühlt. Der ausgefallene orangefarbene Feststoff wurde abfiltriert, mit wenig Toluol gewaschen und im Ölpumpenvakuum getrocknet. Man erhielt 6,3 g (36 %) eines orangefarbenen Pulvers. Das Verhältnis des Racemats zur meso-Form wurde mit 16:1 bestimmt.
¹H-NMR (200 MHz, CDCl₃): 7,7-6,95 (m, 8H, arom. H), 6,55 (s, 2H, Cp-H), 4,85 (s, 2H, CH₂), 2,26 (s, 6H, CH₃₎.
Massenspektrum: 432 M+, korrektes Zerfallsmuster.

### Beispiel 5

### Herstellung von Bis(9-fluorenyl)methan

16,6 g (0,1 mol) Fluoren wurden in 150 ml Toluol gelöst. Anschließend wurden 8,0 g (0,2 mol) Natriumhydroxid und 1,61 g (5 mmol) Tetrabutylammoniumbromid zugegeben. Nach 1 h erfolgte die Zugabe von 1,5 g (0,05 mol) Paraformaldehyd erfolgte portionsweise über einen Zeitraum von 30 min. Nach einer Reaktionszeit von 6 Stunden wurde die Reaktionsmischung mit einer Mischung aus 200 ml Wasser und 12g (0,2 mol) Eisessig hydrolysiert. Die wäßrige Phase wurde abgetrennt und zweimal mit jeweils 100 ml Methylenchlorid extrahiert. Die vereinigten organischen Phasen wurden über MgSO₄ getrocknet, filtriert und im Vakuum auf 50 ml eingeengt. Durch Kühlen auf 0°C und Filtration erhält man 8,5 g (49 %) Bis(9-fluorenyl)methan als farbloses Pulver.
¹H-NMR (200 MHz, CDCl₃): 7,9-6,8 (m, 16H,arom. H), 4,42 (t, 2H, H-Flu), 2,80 (d, 2H, CH₂).
Massenspektrum: 344 M+, korrektes Zerfallsmuster.

### Beispiel 6

### Herstellung von Methylen-(bis-(9-florenyl))-zirkoniumdichlorid

Eine Lösung von 3,44 g (10 mmol) Bis(9-fluorenyl)methan wurden in 70 ml Diethylether gelöst und bei 22°C mit 8 ml (20 mmol) n-Butyllithiumlösung (20 %ig in Toluol) versetzt. Die nach 16 h Rühren erhaltene Suspension wurde auf -30°C gekühlt, dann wurden 2,3 g (10 mmol) Zirkontetrachlorid zugegeben. nach 3 h wurden filtriert, der schwarze Rückstand 4mal mit je 20 ml heißem Toluol gewaschen und im Ölpumpenvakuum getrocknet. Man erhält 4,6 g eines in allen gängigen organischen Lösungsmitteln unlöslichen grünen Pulvers, das im wesentlichen aus Methylen-(bis-(9-florenyl))-zirkoniumdichlorid und Lithiumchlorid besteht.
Massenspektrum: 502 M+, korrektes Zerfallsmuster.

### Beispiel 7

### Polymerisation

Eine Lösung von 10 mg (0,023 mmol) Methylen-(bis-1-(2-methylindenyl))-zirkoniumdichlorid wird mit 1 cm³ 30%iger (4,81 mmol) Methylaluminoxanlösung in Toluol suspendiert.
Parallel dazu wurde ein trockener 16-dm³-Reaktor zunächst mit Stickstoff und anschließend mit Propylen gespült und mit 10 dm³ flüssigem Propylen befüllt.
Dann wurden 3 cm³ Triisobutylaluminium (pur, 12 mmol) mit 30 cm³ Hexan verdünnt in den Reaktor gegeben und der Ansatz bei 30°C 15 Minuten gerührt. Anschließend wurde die Katalysator-Suspension in den Reaktor gegeben. Das Reaktionsgemisch wurde auf die Polymerisationstemperatur von 50°C (bzw. 70°C) aufgeheizt (4°C/min) und das Polymerisationssystem 1 h durch Kühlung bei 50°C (bzw. 70°C) gehalten. Gestoppt wurde die Polymerisation durch Abgasen des restlichen Propylens. Das Polymer wurde im Vakuumtrockenschrank getrocknet und es wies folgende Eigenschaften auf:
Polymerisation bei
   50°C: 30 kg PP/gMet xh, VZ: 14 cm³/g, mp: 106°C, Mw: 6530 g/mol, Mw/Mn: 2.0
Polymerisation bei
   70°C: 68 kg PP/gMet xh, VZ: 14 cm³/g, mp: 100°C, Mw: 6140 g/mol, Mw/Mn: 2.0.

## Patentansprüche

1. Bisindenyl-Verbindung der Formel Methylen-bis(1-Ind)M¹R¹¹R¹², worin
Ind eine Indenylgruppe ist, die einen oder mehrere C₁-C₂₀-Kohlenwasserstoffrest trägt, die auch ein Ringsystem bilden können,
M¹ ein Element der Gruppen IIIb, IVb, Vb oder Vlb des Periodensystems der Elemente ist und
R¹¹ und R¹² gleich oder verschieden sind und ein Wasserstoffatom, ein Halogenatom oder einen C₁-C₄₀-kohlenstoffhaltigen Rest bedeuten.

2. Bisindenyl-Verbindung gemäß Anspruch 1, worin die C₁-C₂₀-Kohlenwasserstoffreste an der Indenylgruppe C₁-C₂₀-Alkyl, C₆-C₂₀-Aryl, C₆-C₂₀-Alkylaryl, C₆-C₂₀-Arylalky oder C₂-C₂₀-Alkenyl sind.

3. Bisindenyl-Verbindung gemäß Anspruch 1 oder 2, worin Ind 2-Methylindenyl, 2-Ethylindenyl, 3-Methylindenyl, 3-Tert.butylindenyl, 3-Trimethylsilylindenyl, 2-Methyl-4-phenylindenyl, 2-Ethyl-4-phenylindenyl, 2-Methyl-4-naphthylindenyl, 2-Methyl-4-isopropylindenyl, Benzoindenyl, 2-Methyl-4,5-benzoindenyl, 2-Methyl-α-acenaphtylindenyl oder 2-Methyl-4,6-diisopropylindenyl bedeutet.

4. Bisindenyl-Verbindung gemäß den Ansprüchen 1 bis 3, worin M¹ Titan, Zirkonium oder Hafnium ist.

5. Bisindenyl-Verbindung gemäß den Ansprüchen 1 bis 4, worin R¹¹ und R¹² C₁-C₄-Alky oder Chlor sind.

6. Katalysator enthaltend a) mindestens eine Bisindenyl-Verbindung gemäß den Ansprüchen 1 bis 5 und b) mindestens einen Cokatalysator.

7. Katalysator gemäß Anspruch 6, wobei der Cokatalysator ein Aluminoxan oder eine Borverbindung ist.

8. Katalysator gemäß Anspruch 6 oder 7, wobei der Katalysator geträgert ist.

9. Verfahren zur Herstellung eines Olefinpolymers durch Polymerisation eines oder mehrerer Olefine in Gegenwart eines Katalysators gemäß den Ansprüchen 6 bis 8.

10. Verfahren gemäß Anspruch 9, wobei als Olefine Ethylen, Propylen, 1-Buten, 1-Hexen, 1-Octen, 4-Methyl-1-penten, 1,3-Butadien, Isopren, Norbornen, Dimethanooctahydonaphthalin oder Norbornadien eingesetzt werden.
